# EUROPÄISCHE PATENTSCHRIFT

(11) **EP 0 242 842 B1**
(45) Veröffentlichungstag und Bekanntmachung des Hinweises auf die Patenterteilung: **03.03.1993**
(21) Anmeldenummer: 87105822.8
(22) Anmeldetag: 21.04.1987
(51) Int. Cl.: A61B 17/58

(54) **Knochenplattenanordnung**
Bone plate arrangement
Dispositif de plaque osseuse

(30) Priorität: 21.04.1986 DE 8610858 U
(43) Veröffentlichungstag der Anmeldung: 28.10.1987
(73) Patentinhaber: Wolter, Dietmar, Prof. Dr., D-21033 Hamburg (DE)
(72) Erfinder: Wolter, Dietmar, Prof. Dr., D-2000 Hamburg 1 (DE)
(74) Vertreter: Glawe, Delfs, Moll & Partner

(56) Entgegenhaltungen:
- EP-A- 0 053 999
- EP-A- 0 201 024
- US-A- 3 779 240

## Beschreibung

Die Erfindung bezieht sich auf eine Knochenplattenanordnung bestehend aus einer Knochenplatte und wenigstens einer Knochenschraube, wobei die Knochenplatte eine Durchgangsöffnung zur richtungsunbestimmten Aufnahme des Schafts der Knochenschraube aufweist und beide Teile formentsprechend zusammenwirkende Auflageflächen bilden.

Die zusammenwirkenden Auflageflächen von Knochenplatten und Knochenschrauben werden im allgemeinen winkelbeweglich ausgeführt, insbesondere mit einer sphärischen Auflagefläche des Schraubenkopfes. Dadurch soll bewirkt werden, daß die Befestigung der Knochenplatte am Knochen unabhängig von der Knochenschraubenrichtung zustande kommt, die von der zur Knochenplatte lotrechten Richtung zufällig oder beabsichtigt zur Berücksichtigung anatomischer Bedingungen abweichen kann (DE-PS 29 38 202).

Bekannt sind auch Knochenschrauben mit ebener Ausführung der Auflageflächen am Schraubenkopf und an der Auflageplatte (DE-OS 28 32 555, Fig.2), die aber nur für untergeordnete Anwendungszwecke verwendet werden, wenn es entweder bei im Knochen festgelegter Schraubenrichtung auf eine genaue gegenseitige Anlage der Auflageflächen nicht ankommt oder ein kurzer Schraubenschaft im weichen Knochenmaterial sich ohne Nachteil beim Festziehen so schwenken läßt, daß seine Richtung sich auf die zu den Auflageflächen koaxiale Richtung einstellt.

Die Richtung des mit der Knochenplatte verschraubten Knochenfragments wird in jedem Fall dadurch bestimmt, daß die Oberfläche des Knochenfragments an die Oberfläche der Knochenplatte durch die Verschraubung angepreßt wird und dadurch mit dieser richtungsgleich verlaufen muß.

Der Erfindung liegt die Aufgabe zugrunde, die Anwendungsmöglichkeiten von Knochenplatten zu erweitern.

Die erfindungsgemäße Lösung besteht darin, daß die Auflageflächen richtungsbestimmend in bezug auf eine von der zur Knochenplatte lotrechten Richtung abweichenden Richtung ausgebildet sind. Vorzugsweise sind die Auflageflächen eben oder übereinstimmend konisch.

Die Erfindung gibt die Möglichkeit, dem mit der Knochenplatte verschraubten Knochenfragment eine Richtung zu erteilen, die durch die Richtung der Knochenschraube und der Auflageflächen vorherbestimmt ist und nicht unbedingt abhängig sein muß von der übereinstimmenden Richtung der Oberfläche des Knochenfragments und der Oberfläche der Knochenplatte. Die Erfidnung erlaubt es demnach, auch solchen Knochenfragmenten eine vorbestimmte Richtung aufzuzwingen, die keine einfache, mit der Knochenplatte in Übereinstimmung bringbare Oberflächengestalt aufweisen oder an die die Knochenplatte nicht durch Biegung anformbar ist.

Die Erfindung eröffnet die Möglichkeit zu einem Operationsverfahren, bei dem die Richtung eines Knochenfragments während der Anbringung der Knochenplatte gezielt verändert wird. Zu diesem Zweck wird zunächst das Schraubenloch im allgemeinen lotrecht zur Knochenplatte vorbereitet und die Knochenschraube in diese Richtung eingedreht. Wenn gegen Ende des Eindrehens der Schraube die Auflageflächen zusammenkommen, suchen sie sich unter der Schraubspannung formgleich aneinander anzulegen, wodurch die Schraube verschwenkt wird, bis sie achsgleich mit den Auflageflächen verläuft.

Die Erreichung und Aufrechterhaltung der gewünschten Richtung ist damit abhängig von einer engen Anlage der Auflageflächen aneinander. Im allgemeinen wird diese bei der Operation durch die Schraubspannung erreicht. Jedoch gibt es Fälle, in denen die Belastung des Knochenfragments mit der für sichere Anlage der Auflageflächen aneinander erforderlichen Schraubspannung unerwünscht ist oder in denen die Anlage durch die Schraubspannung allein auf die Dauer nicht gewährleistet werden kann. Für diese Fälle ist es zweckmäßig, die Knochenplatte mit einer Deckplatte zu versehen, die mit ihr unter Pressung auf den Kopf der Knochenschraube verbindbar ist.

Die Erfindung wird im folgenden näher unter Bezugnahme auf die Zeichnung erläutert, die ein vorteilhaftes Ausführungsbeispiel veranschaulicht. Darin zeigen:

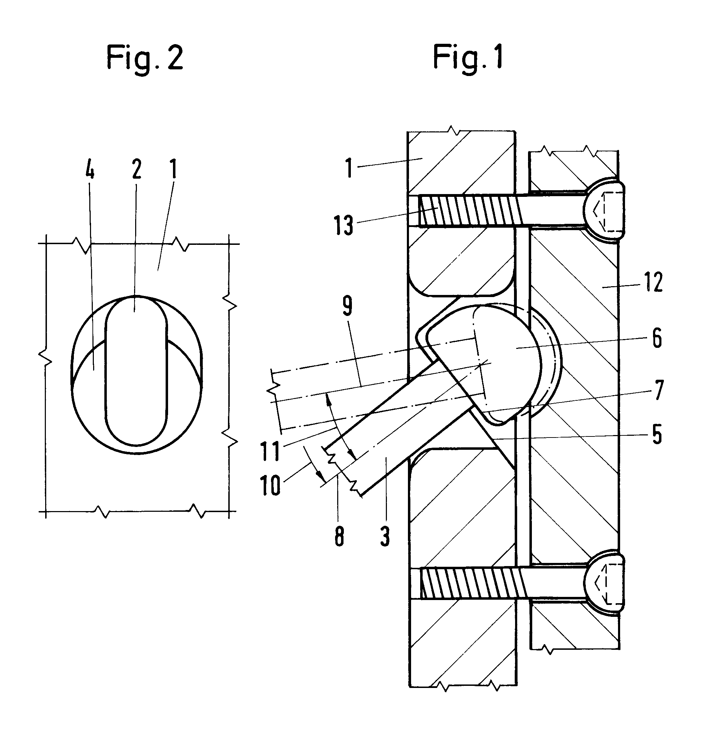

- Fig. 1: einen vergrößerten Teil-Längsschnitt durch eine Knochenplattenanordnung,
- Fig. 2: eine Draufsicht auf den in Fig.1 dargestellten Knochenplattenteil und
- Fig. 3: eine Seitenansicht einer Knochenplattenanordnung in Anwendung auf drei Wirbelkörper.

Die Knochenplatte 1 enthält eine Durchgangsöffnung 2, die als Langloch in Längsrichtung der Knochenplatte ausgeführt ist, um in dieser Richtung dem Schaft 3 einer Knochenschraube keine Winkelbeschränkungen aufzuerlegen. Im Umkreis der Durchgangsöffnung 2 enthält die Knochenplatte eine Einsenkung 4 zur Bildung einer Auflagefläche 5, die gegenüber der Längsrichtung der Knochenplatte geneigt und eben ist.

Der zu dem Knochenschraubenschaft 3 gehörige Schraubenkopf 6 weist eine unterseitige Auflagefläche 7 auf, die gleichfalls eben und lotrecht zur Schraubenlängsachse 8 ausgeführt ist.

Wird die Knochenschraube in der gestrichelt angedeuteten Weise in der Richtung 9, die etwa lotrecht zur Platte verläuft (aber nicht verlaufen muß) in ein Knochenfragment eingeschraubt, so wird sich in der Endphase des Schraubvorgangs unter der Einwirkung der in der Längsachse der Schraube wirkenden Schraubspannung die Auflagefläche 7 des Schraubenkopfs an die Auflagefläche 5 der Platte anlegen wollen, so daß eine Verschwenkung im Sinne des Pfeils 10 zustande kommt, bis vollständige Anlage der Auflageflächen aneinander erreicht ist. Dadurch läßt sich eine Verschwenkung des den Schaft 3 der Schraube aufnehmenden Knochenfragments um den Winkel 11 erreichen.

Wenn es untunlich erscheint, die für die Verschwenkung erforderliche Schraubspannung auf das Knochenfragment aufzuüben oder wenn wegen der Gestaltung der Oberfläche des Fragments aus geometrischen Gründen die Verschwenkung nicht möglich ist, oder wenn eine Aufrechterhaltung der gewünschten Winkellage aufgrund der Schraubspannung allein nicht gewährleistet ist, bedient man sich der zur Knochenplatte 1 gehörigen Deckplatte 12, die mittels der Schrauben 13 derart gegen den Schraubenkopf 6 gepreßt wird, daß die Anlageflächen 5, 7 mit Sicherheit zusammengehalten werden.

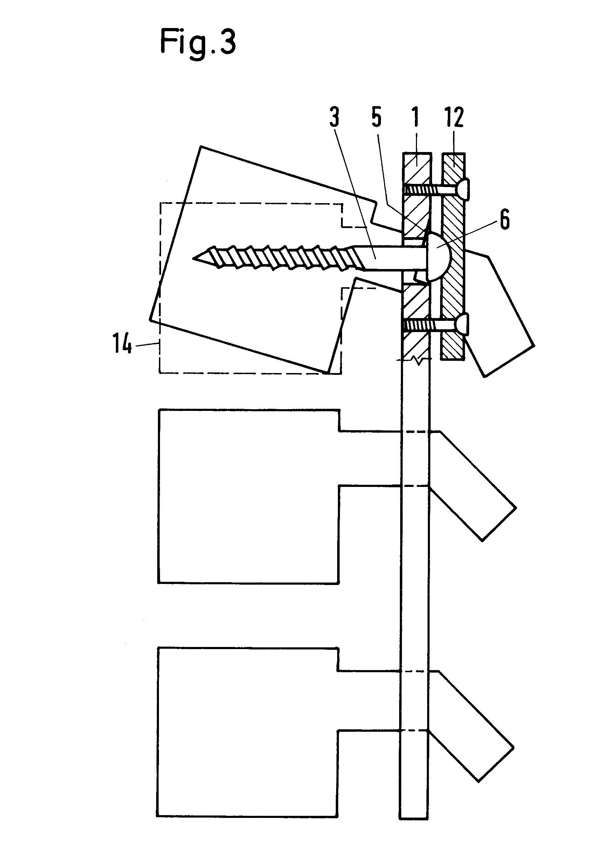

Im Anwendungsbeispiel gemäß Fig. 3 stellt sich das Problem, den Wirbelkörper 14 aus der mit gestrichelten Linien dargestellten Stellung, die er infolge eines Unfalls eingenommen hat, aufzurichten in die mit durchgezogenen Linien dargestellte, anatomisch richtige Lage. Zwei Knochenplatten 1 werden in symmetrischer Anordnung beiderseits der Dornfortsätze mit den dargestellten Wirbelkörpern verschraubt, wobei die Knochenplatte im Bereich des aufzurichtenden Wirbelkörpers 14 eine geneigte Auflagefläche 5 aufweist, deren Winkel in bezug auf die Längsrichtung der Knochenplatte etwa dem gewünschten Neigungswinkel des aufzurichtenden Wirbelkörpers gegenüber den benachbarten Wirbelkörpern entspricht. Werden nun die Knochenschrauben parallel zur Hauptrichtung des Wirbelkörpers durch die Wirbelbogenansätzeneben dem Wirbelkanal in den Wirbelkörper eingedreht, so richtet sich dieser bei der Anlage des Schraubenkopfs 6 an der Auflagefläche 5 in der gewünschten Weise auf, was durch die Deckplatte 12 unterstützt und gesichert werden kann.

## Patentansprüche

1. Knochenplattenanordnung bestehend aus einer Knochenplatte (1) und wenigstens einer Knochenschraube, wobei die Knochenplatte (1) eine Durchgangsöffnung (2) zur richtungsunbestimmten Aufnahme des Schafts (3) der Knochenschraube aufweist und beide Teile formentsprechend zusammenwirkende Auflageflächen (5,7) bilden, dadurch gekennzeichnet, daß die Auflageflächen (5,7) richtungsbestimmend in bezug auf eine von der zur Knochenplatte (1) lotrechten Richtung (9) abweichende Richtung (8) ausgebildet sind.

2. Knochenplattenanordnung nach Anspruch 1, dadurch gekennzeichnet, daß die Auflageflächen (5,7) eben sind.

3. Knochenplattenanordnung nach Anspruch 1, dadurch gekennzeichnet, daß die Auflageflächen übereinstimmend konisch sind.

4. Knochenplattenanordnung nach einem der Ansprüche 1 bis 3, dadurch gekennzeichnet, daß die Knochenplatte mit einer Deckplatte (12) versehen ist, die mit ihr unter Pressung auf den Kopf (6) der Knochenschraube verschraubbar ist.

## Claims

1. A bone plate assembly, comprising a bone plate (1) and at least one bone screw, wherein the bone plate (1) has an opening (2) for accommodating in a directionally indeterminate manner the shank (3) of the bone screw, and both parts form support surfaces (5, 7) which co-operate in a shape-conforming manner, characterised in that the support surfaces (5, 7) are formed in a directionally determinate manner in relation to a direction (8) diverging from the direction (9) perpendicular to the bone plate (1).

2. A bone plate assembly according to Claim 1, characterised in that the support surfaces (5, 7) are flat.

3. A bone plate assembly according to Claim 1, characterised in that the support surfaces are correspondingly conical.

4. A bone plate assembly according to any one of Claims 1 to 3, characterised in that the bone plate is provided with a cover plate (12) which can be screwed thereto while pressing on the head (6) of the bone screw.

## Revendications

1. Système de plaque osseuse, se composant d'une plaque osseuse (1) et d'au moins une vis à os, la plaque osseuse (1) présentant une ouverture de passage (2) destinée à recevoir sans direction déterminée la tige (3) de la vis à os et les deux éléments formant des surfaces d'appui (5, 7) qui coopèrent par coïncidence de forme, caractérisé en ce que les surfaces d'appui (5, 7) sont réalisées de façon à déterminer une direction (8) qui s'écarte de la direction (9) perpendiculaire à la plaque osseuse (1).

2. Système de plaque osseuse selon la revendication 1, caractérisé en ce que les surfaces d'appui (5, 7) sont planes.

3. Système de plaque osseuse selon la revendication 1, caractérisé en ce que les surfaces d'appui ont des formes coniques correspondantes.

4. Système de plaque osseuse selon l'une quelconque des revendications 1 à 3, caractérisé en ce que la plaque osseuse est munie d'une plaque de recouvrement (12) qui peut être vissée sur elle en exerçant une pression sur la tête (6) de la vis à os.
